# EUROPEAN PATENT SPECIFICATION

(11) **EP 4 135 816 B1**
(45) Date of publication and mention of the grant of the patent: **27.05.2026**
(21) Application number: 21719366.3
(22) Date of filing: 23.03.2021
(51) Int. Cl.: A61M 25/00, A61M 25/06, A61M 39/10

(54) **CATHETER ADAPTER PUSH TAB AND RELATED DEVICES, SYSTEMS**
KATHETERADAPTERSCHUBLASCHE UND ZUGEHÖRIGE VORRICHTUNGEN, SYSTEME
POUSSOIR D'ADAPTATEUR DE CATHÉTER, ET DISPOSITIFS ET SYSTÈMES ASSOCIÉS

(30) Priority: 13.04.2020 US 202063009350 P; 22.03.2021 US 202117208994
(43) Date of publication of application: 22.02.2023
(73) Proprietor: Becton, Dickinson and Company, Franklin Lakes, NJ 07417 (US)
(72) Inventor: WONG, Yun Hui, Fernvale 791408 (SG); CHENG, Kiat Jin, Bishan 570125 (SG)
(74) Representative: dompatent
(86) International application number: PCT/US2021/023654
(87) International publication number: WO 2021/211268

(56) References cited:
- US-A1- 2004 102 735
- US-A1- 2017 296 782
- US-A1- 2020 038 634
- US-S- D 837 368

## Description

### BACKGROUND

Infusion therapy, a common healthcare procedure, may be facilitated by a vascular access device. Hospitalized, home care, and other patients receive fluids, pharmaceuticals, and blood products via a vascular access device inserted into the vascular system. Blood withdrawal is another common healthcare procedure that may be facilitated by the vascular access device.

The vascular access device may access a peripheral or central vasculature of a patient. The vascular access device may be indwelling for short term (days), moderate term (weeks), or long term (months to years). The vascular access device may be used for continuous infusion therapy or for intermittent therapy.

A common type vascular access device is an over-the-needle peripheral intravenous catheter (PIVC). As its name implies, the "over-the-needle" PIVC may be mounted over an introducer needle having a sharp distal tip. The sharp distal tip may be used to pierce skin and the vasculature of the patient. Insertion of the PIVC into the vasculature may follow the piercing of the vasculature by the introducer needle. The introducer needle and the PIVC are generally inserted at a shallow angle through the skin into the vasculature of the patient with a bevel of the introducer needle facing away from the skin of the patient. Once placement of the introducer needle within the vasculature has been confirmed, the clinician may withdraw the introducer needle from the PIVC, leaving the PIVC in place for future fluid infusion and/or blood withdrawal.

The PIVC may extend from a catheter adapter, which may include a push tab to aid the clinician in inserting the PIVC into the vasculature. In some instances, securement tape may be used to secure the catheter adapter to skin of the patient. However, the push tab may result in tenting of the securement tape, which may result in improper securement of the catheter adapter to the patient and possible dislodgement.

Prior art has attempted to address the issue of tenting and securement of the catheter adapter. For example, US2004102735A1, US2017296782A1, and US2004102735A1 disclose a high push tab that is unfoldable, which may provide a better grip for insertion but may still interfere with the securement tape, leading to possible dislodgement. On the other hand, USD837368S1 describes a flat push tab that does not interfere with the securement tape, but its flat design may not offer the same amount of grip for the clinician during insertion. Therefore, there remains a need for an improved catheter adapter design that provides both a secure grip for insertion and proper securement without causing tenting of the securement tape.

The subject matter claimed herein is not limited to embodiments that solve any disadvantages or that operate only in environments such as those described above. Rather, this background is only provided to illustrate one example technology area where some implementations described herein may be practiced.

### SUMMARY

The invention is defined in independent claims 1 and 9. Further aspects are defined in dependent claims 2-8 and 10, 11. Methods of treatment or use do not form part of the claimed invention and are for illustrative purposes.

The present disclosure relates generally to a catheter adapter push tab, as well as related devices, systems, and methods. In some embodiments, a catheter assembly may include a catheter adapter, which may include a distal end, a proximal end, and a lumen extending between the distal end and the proximal end. In some embodiments, the catheter assembly may include a push tab, which may be pivotally coupled to the catheter adapter. In some embodiments, the push tab may be pivotally moveable between a raised position and a depressed position.

In some embodiments, the push tab may include a first end and a second end opposite the first end. In some embodiments, the first end may be pivotally coupled to the catheter adapter. In some embodiments, the second end may be further from the catheter adapter when the push tab is in the raised position than when the push tab is in the depressed position.

In some embodiments, the second end may be disposed in a first position in response to the push tab being in the raised position. In some embodiments, the second end may be disposed in a second position in response to the push tab being in the depressed position. In some embodiments, the second position may be proximal to the first position.

In some embodiments, the push tab may include two opposing protrusions. In some embodiments, the catheter adapter may include two opposing indents. In some embodiments, the two opposing protrusions may be disposed within the two opposing indents to pivotally couple the push tab to the catheter adapter.

In some embodiments, in response to the push tab being in the raised position, the push tab may be perpendicular or generally perpendicular to a longitudinal axis of the catheter adapter. In some embodiments, in response to the push tab being in the depressed position, the push tab may be generally aligned with the longitudinal axis of the catheter adapter. In some embodiments, the push tab may include an edge configured to contact the catheter adapter in response to the push tab pivotally moving from the depressed position to the raised position.

In some embodiments, a proximal surface of the push tab may be concave, which may facilitate securement of a digit of the clinician against the proximal surface. In some embodiments, the catheter adapter may include a side port disposed between the distal end and the proximal end of the catheter adapter. In some embodiments, the push tab may be pivotally coupled to the catheter adapter proximal to the side port.

In some embodiments, the catheter adapter may include a resilient push tab, which may be coupled to the catheter adapter. In some embodiments, the resilient push tab may include a first end, a second end, and an arced portion between the first end and the second end. In some embodiments, the resilient push tab may be configured to move from a raised position to a depressed position. In some embodiments, the arced portion may be further from the catheter adapter when the resilient push tab is in the raised position than when the resilient push tab is in the depressed position. In some embodiments, in response to the resilient push tab being in the raised position, the first end and the second end may be biased outwardly by the catheter adapter. In some embodiments, in response to the resilient push tab moving from the raised position to the depressed position, the first end and the second end may be brought closer together.

In some embodiments, the arced portion may contact the catheter adapter in response to the resilient push tab being moved from the raised position to the depressed position. In some embodiments, the resilient push tab may be coupled to the catheter adapter proximal to the side port.

In some embodiments, a method (not claimed) may include inserting a catheter assembly into vasculature of the patient. In some embodiments, the catheter assembly may include the catheter adapter, which may include the distal end, the proximal end, and the lumen. In some embodiments, the catheter assembly may include a push tab coupled to the catheter adapter. In some embodiments, the push tab may be moveable between a first position and a second position. In some embodiments, the push tab may be disposed in the first position.

In some embodiments, when the catheter assembly is inserted into the vasculature of the patient, the method may include pushing down on the push tab to move the push tab from the first position to the second position. In some embodiments, after pushing down on the push tab to move the push tab from the first position to the second position, the method may include applying securement tape over the push tab with the push tab in the second position.

In some embodiments, the push tab may be pivotally coupled to the catheter adapter. In some embodiments, the push tab may be pivotally moveable between the first position and the second position. In some embodiments, the first position may include the raised position. In some embodiments, the second position may include the depressed position. In some embodiments, the push tab may include the resilient push tab. In some embodiments, the arced portion may be further from the catheter adapter when the resilient push tab is in the first position than when the resilient push tab is in the second position.

It is to be understood that both the foregoing general description and the following detailed description are exemplary and explanatory and are not restrictive of the invention, as claimed. It should be understood that the various embodiments are not limited to the arrangements and instrumentality shown in the drawings. It should also be understood that the embodiments may be combined, or that other embodiments may be utilized and that structural changes, unless so claimed, may be made without departing from the scope of the various embodiments of the present invention. The following detailed description is, therefore, not to be taken in a limiting sense.

### BRIEF DESCRIPTION OF THE SEVERAL VIEWS OF THE DRAWINGS

Example embodiments will be described and explained with additional specificity and detail through the use of the accompanying drawings in which:
Figure 1A is an upper perspective view of an example catheter system, illustrating an example push tab in a raised position, according to some embodiments;
Figure 1B is another upper perspective view of the catheter system, illustrating a digit of a clinician pushing on the push tab of Figure 1A in the raised position during insertion of the catheter system into vasculature of the patient, according to some embodiments;
Figure 1C is another upper perspective view of the catheter system, illustrating the push tab of Figure 1A in the raised position, according to some embodiments;
Figure 1D is an upper perspective view of the catheter system, illustrating an example needle assembly partially removed from an example catheter assembly, and the push tab of Figure 1A in a depressed position, according to some embodiments;
Figure 2A is a perspective view of the push tab of Figure 1A, according to some embodiments;
Figure 2B is another perspective view of the push tab of Figure 1A, according to some embodiments;
Figure 3A is an upper perspective view of the catheter assembly of the catheter system, illustrating the push tab of Figure 1A removed for illustrative purposes, according to some embodiments;
Figure 3B is an upper perspective view of the catheter assembly of the catheter system, illustrating the push tab of Figure 1A removed for illustrative purposes, according to some embodiments;
Figure 4A is an upper perspective view of the catheter system, illustrating another push tab disposed in a raised position, according to some embodiments;
Figure 4B is an upper perspective view of the catheter assembly, illustrating the other push tab disposed in a depressed position, according to some embodiments;
Figure 4C is an end view of the other push tab, according to some embodiments;
Figure 4D is a proximal end view of the catheter assembly, illustrating the other push tab in the raised position; and
Figure 4E is a proximal end view of the catheter assembly, illustrating the other push tab in the depressed position, according to some embodiments.

### DESCRIPTION OF EMBODIMENTS

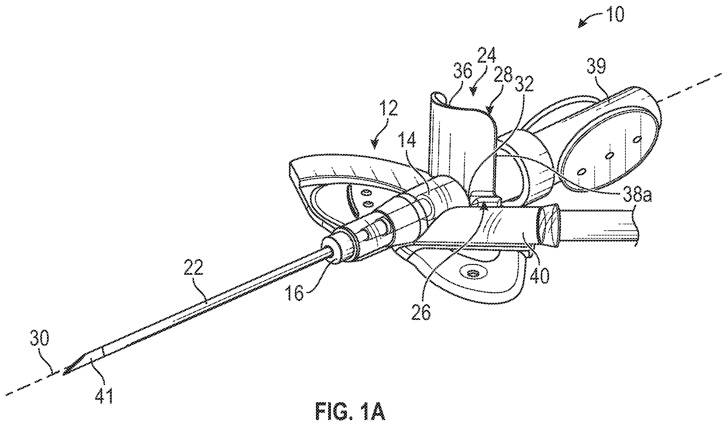

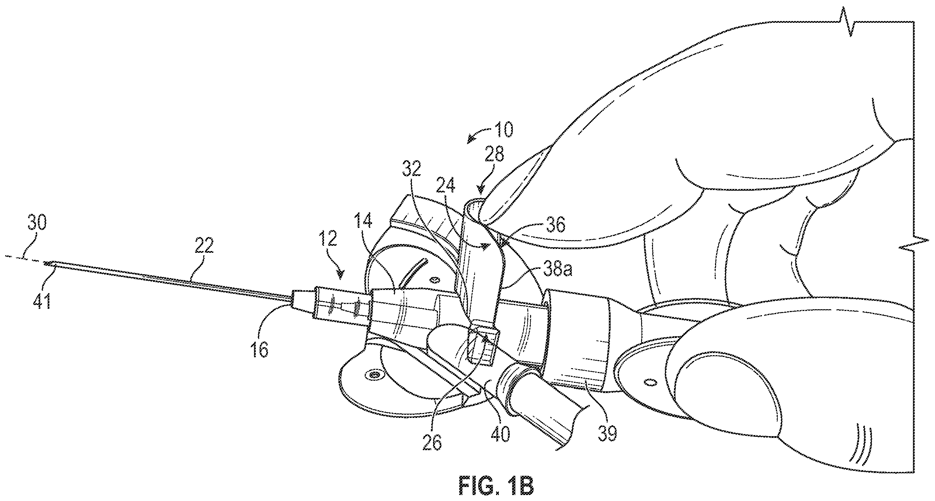

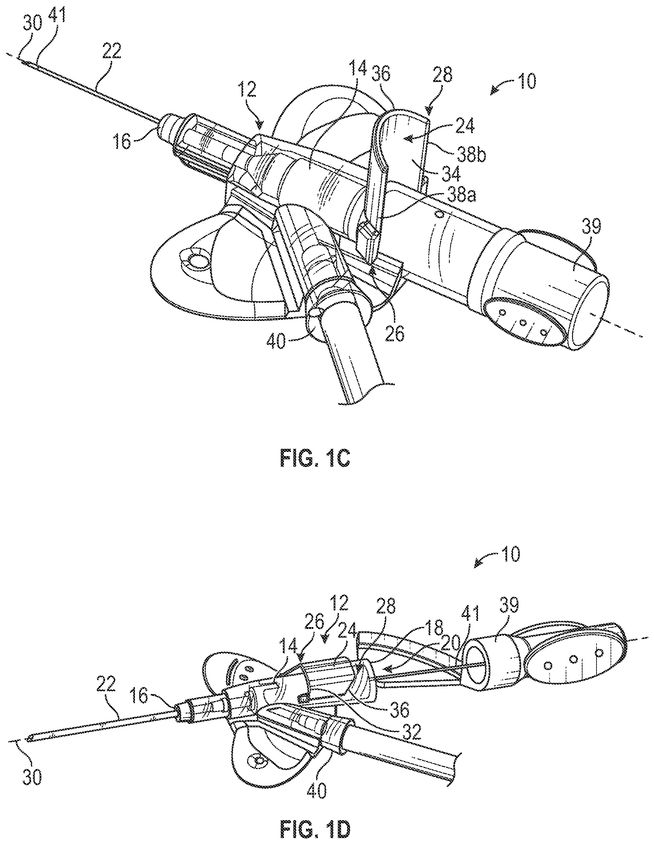

Referring now to Figures 1A-1D, an example catheter system 10 is illustrated, according to some embodiments. In some embodiments, the catheter system 10 may include a catheter assembly 12, which may include a catheter adapter 14. In some embodiments, the catheter adapter 14 may include a distal end 16, a proximal end 18, and a lumen 20 extending between the distal end 16 and the proximal end 18. In some embodiments, the catheter assembly 12 may include a catheter 22, which may be secured within the catheter adapter 14 and extend distally from the distal end 16 of the catheter adapter 14. In some embodiments, the catheter 22 may include a peripheral intravenous catheter (PIVC), a peripherally-inserted central catheter, or a midline catheter.

In some embodiments, the catheter assembly 12 may include a push tab 24, which may be pivotally coupled to the catheter adapter 14. In some embodiments, the push tab 24 may be pivotally moveable between a raised position, illustrated, for example, in Figures 1A-1C, and a collapsed or depressed position, illustrated, for example, in Figure 1D.

In some embodiments, the push tab 24 may include a first end 26 and a second end 28 opposite the first end 26. In some embodiments, the first end 26 may be pivotally coupled to the catheter adapter 14. In some embodiments, the second end 28 may be further from the catheter adapter 14 when the push tab 24 is in the raised position than when the push tab 24 is in the depressed position.

In some embodiments, the second end 28 may be disposed in a first position in response to the push tab 24 being in the raised position, as illustrated, for example, in Figures 1A-1C. In some embodiments, the second end 28 may be disposed in a second position in response to the push tab 24 being in the depressed position, as illustrated, for example, in Figure 1D. In some embodiments, the second position of the second end 28 may be proximal to the first position of the second end 28.

In some embodiments, in response to the push tab 24 being in the raised position, the push tab 24 may be perpendicular or generally perpendicular to a longitudinal axis 30 of the catheter adapter 14. For example, the push tab 24 may be angled between 45 degrees and 135 degrees or between 60 degrees and 120 degrees with respect to the longitudinal axis 30. In some embodiments, the push tab 24 be angled at 90 degrees with respect to the longitudinal axis 30, which may facilitate pushing by a digit of the clinician. In some embodiments, in response to the push tab 24 being in the depressed position, the push tab 24 may be aligned or generally aligned with the longitudinal axis 30 of the catheter adapter 14. In some embodiments, the push tab 24 may include an edge 32 configured to contact the catheter adapter 14 in response to the push tab 24 pivotally moving from the depressed position to the raised position. In some embodiments, the edge 32 may contact the catheter adapter 14 to prevent the push tab 24 from moving distal to the raised position. In some embodiments, the edge 32 may be arc-shaped.

In some embodiments, a proximal surface 34 of the push tab 24 may be concave, which may facilitate securement of a digit of the clinician against the proximal surface 34. In some embodiments, the proximal surface 34 may be bordered by the edge 32, another edge 36 opposite the edge 32, and two generally parallel edges 38a,b extending between the edge 32 and the other edge 36,

In some embodiments, the catheter adapter 14 may include a side port 40 disposed between the distal end 16 and the proximal end 18 of the catheter adapter 14. In some embodiments, the push tab 24 may be pivotally coupled to the catheter adapter 14 proximal to the side port 40. In some embodiments, an extension tube may be integrated with the side port 40. In some embodiments, the side port '40 may be used for blood collection and/or fluid infusion. In some embodiments, the catheter adapter 14 may not include the side port 40.

In some embodiments, the push tab 24 may be positioned to facilitate one-handed insertion by the clinician into the vasculature of the patient. In some embodiments, a length of the push tab 24 between the first end 26 and the second end 28 may also be great enough to facilitate one-handed insertion by the clinician into the vasculature of the patient.

In some embodiments, the catheter system 10 may include a needle assembly, which may include a needle hub 39 and an introducer needle 41 extending through the catheter 22. In some embodiments, a proximal end of the introducer needle 41 may be secured within the needle hub 39. In some embodiments, the introducer needle 41 may include a sharp distal tip. In some embodiments, the needle hub 39 may include opposite side grips, which may facilitate the one-handed grip. In some embodiments, the needle assembly may be removed from the catheter system 10 in response to the catheter 22 being placed within the vasculature.

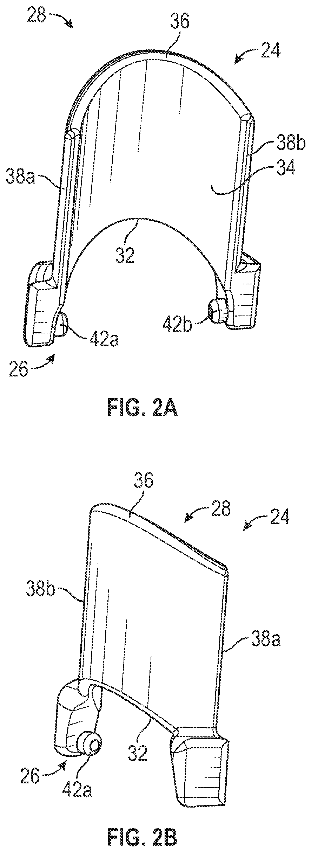

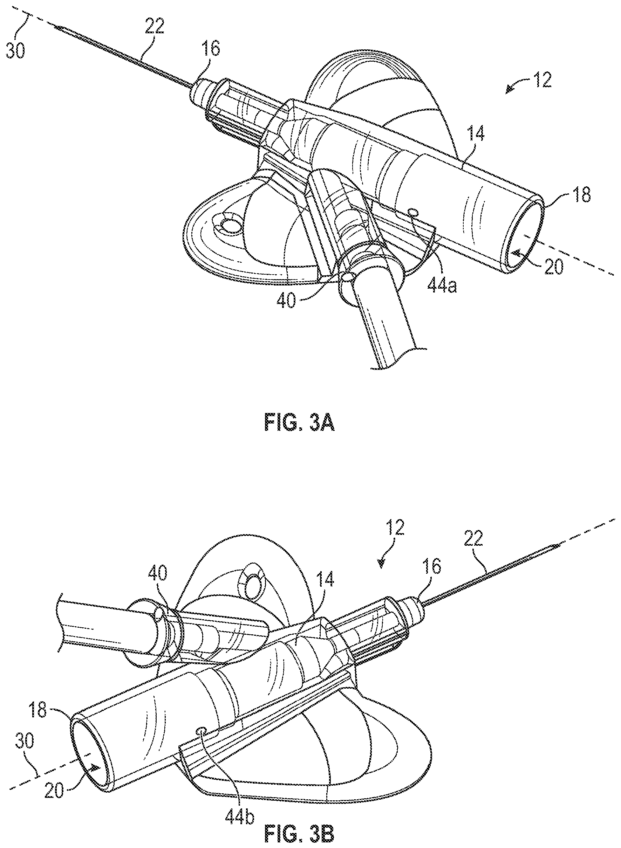

Referring now to Figures 2A-2B, in some embodiments, the push tab 24 may include two opposing protrusions 42a,b. Referring now to Figures 3A-2B, in some embodiments, the catheter adapter may include two opposing indents 44a,b. In some embodiments, the two opposing protrusions 42a,b may be disposed within the two opposing indents 44a,b to pivotally couple the push tab 24 to the catheter adapter 14.

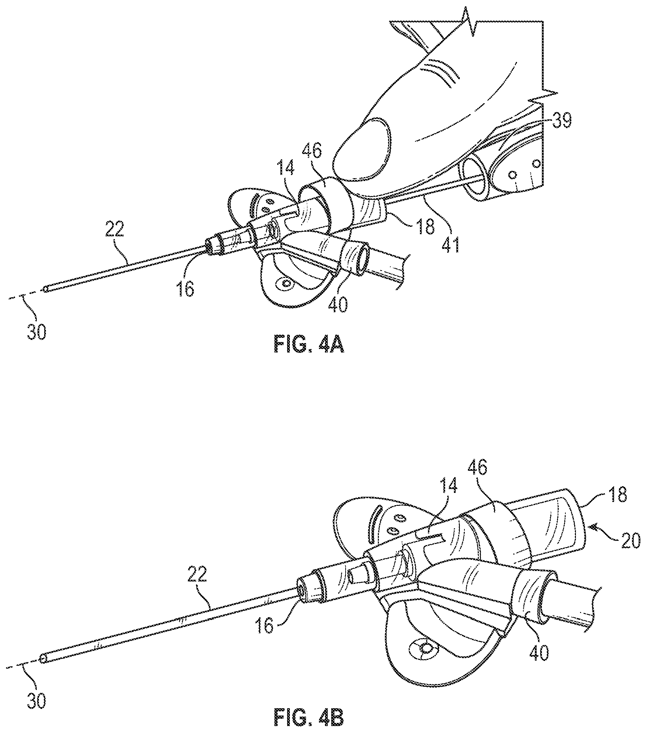

Referring now to Figures 4A-4E, in some embodiments, the catheter adapter 14 may include a resilient push tab 46, which may be coupled to the catheter adapter 14. In some embodiments, the resilient push tab 46 may include a first end 48, a second end 50, and an arced portion 52 between the first end 48 and the second end 50. In some embodiments, the resilient push tab 46 may be constructed of metal, an elastomer, or another suitable material.

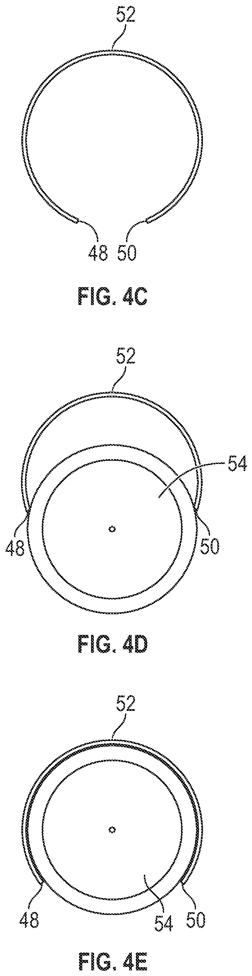

In some embodiments, the resilient push tab 46 may be configured to move from a raised position, illustrated for example, in Figures 4A and 4D, to a depressed position, illustrated, for example in Figures 4B and 4E. In some embodiments, the arced portion 52 may be further from the catheter adapter 14 when the resilient push tab 46 is in the raised position than when the resilient push tab 46 is in the depressed position. In some embodiments, in response to the resilient push tab 46 being in the raised position, the first end 48 and the second end 50 may be biased outwardly from a resting position, illustrated, for example, in Figure 4C, by the catheter adapter 14. In some embodiments, in response to the resilient push tab 46 moving from the raised position to the depressed position, the first end 48 and the second end 50 may be brought closer together and an inner diameter of the resilient push tab 46 may decrease.

In some embodiments, the arced portion 52 may contact the catheter adapter 14 in response to the resilient push tab 46 being moved from the raised position to the depressed position. In some embodiments, the resilient push tab 46 may be coupled to the catheter adapter 14 proximal to the side port 40. In some embodiments, an outer surface of the catheter adapter 14 may include indents that may hold the first end 48 and the second end 50 in the raised position, but the first end 48 and the second end 50 may be configured to be removed from the indents in response to depression and pressing of the resilient push tab 46 by the clinician. In some embodiments, the catheter adapter 14 may not include the indents. In some embodiments, a septum 54 within the catheter adapter 14 may seal the proximal end 18 of the catheter adapter 14.

In some embodiments, a method may include inserting the catheter assembly 12 into vasculature of the patient. In some embodiments, the catheter assembly 12 may include the push tab 24 (discussed with respect to Figures 1A-3B) coupled to the catheter adapter 14. In some embodiments, the catheter assembly 12 may include the resilient push tab 46 coupled to the catheter adapter 14. In some embodiments, the push tab 24 or the resilient push tab 46 may be moveable between a raised position and a second position. In some embodiments, the push tab 24 or the resilient push tab 46 may be disposed in the raised position during insertion of the catheter assembly 12 into the vasculature of the patient. In some embodiments, insertion of the catheter assembly 12 into the vasculature of the patient may include initial insertion through the skin and a wall of a blood vessel and/or include advancement of the catheter 22 distally with respect to the introducer needle 41 in response to the introducer needle 41 being positioned within the vasculature.

In some embodiments, after the catheter assembly 12 is inserted into the vasculature of the patient, the method may include pushing down on the push tab 24 and/or the resilient push tab 46 to move the push tab 24 or the resilient push tab 46 from the raised position to the second position. In some embodiments, after pushing down on the push tab 24 or the resilient push tab 46 to move the push tab 24 or the resilient push tab 46 from the raised position to the second position, the method may include applying securement tape over the push tab 24 or the resilient push tab 46 with the push tab 24 or the resilient push tab 46 in the second position. In some embodiments, applying the securement tape over the push tab 24 or the resilient push tab 46 in the second position may prevent tenting of the securement tape and may increase stability of the catheter assembly 12 with respect to the patient.

It is to be understood that both the foregoing general description and the following detailed description are examples and explanatory and are not restrictive of the invention, as claimed. It should be understood that the various embodiments are not limited to the arrangements and instrumentality shown in the drawings. It should also be understood that the embodiments may be combined, or that other embodiments may be utilized and that structural changes, unless so claimed, may be made without departing from the scope of the various embodiments of the present invention. The following detailed description is, therefore, not to be taken in a limiting sense.

All examples and conditional language recited herein are intended for pedagogical objects to aid the reader in understanding the invention and the concepts contributed by the inventor to furthering the art, and are to be construed as being without limitation to such specifically recited examples and conditions. Although embodiments of the present inventions have been described in detail, it should be understood that the various changes, substitutions, and alterations could be made hereto without departing from the scope of the invention.

## Claims

1. A catheter assembly (12), comprising:
a catheter adapter (14), comprising a distal end (16), a proximal end (18), and a lumen (20) extending between the distal end and the proximal end; and
a push tab (24) comprising:
a first end (26);
a second end (28) opposite the first end; and
a length extending between the first end and the second end;
**CHARACTERIZED IN THAT**
the push tab is pivotally coupled to the catheter adapter, wherein the push tab is pivotally moveable between a raised position and a depressed position, wherein the first end is pivotally coupled to the catheter adapter, wherein the second end is further from the catheter adapter when the push tab is in the raised position than when the push tab is in the depressed position.

2. The catheter assembly of claim 1, wherein the second end is disposed in a first position in response to the push tab being in the raised position, wherein the second end is disposed in a second position in response to the push tab being in the depressed position, wherein the second position is proximal to the first position.

3. (Currently amended). The catheter assembly of claim 1, wherein the push tab comprises two opposing protrusions (42a,b), wherein the catheter adapter comprises two opposing indents (44a,b), wherein the two opposing protrusions are disposed within the two opposing indents to pivotally couple the push tab to the catheter adapter.

4. (Currently amended). The catheter assembly of claim 1, wherein in response to the push tab being in the raised position, the push tab is generally perpendicular to a longitudinal axis (30) of the catheter adapter.

5. (Currently amended). The catheter assembly of claim 1, wherein in response to the push tab being in the depressed position, the push tab is generally aligned with a longitudinal axis (30) of the catheter adapter.

6. (Currently amended). The catheter assembly of claim 1, wherein the push tab comprises an edge (32) configured to contact the catheter adapter in response to the push tab pivotally moving from the depressed position to the raised position.

7. (Currently amended). The catheter assembly of claim 1, wherein a proximal surface 34 of the push tab is concave.

8. (Currently amended). The catheter assembly of claim 1, wherein the catheter adapter comprises a side port (40) disposed between the distal end and the proximal end, wherein the push tab is pivotally coupled to the catheter adapter proximal to the side port.

9. A catheter assembly (12), comprising:
a catheter adapter (14), comprising a distal end (16), a proximal end (18), and a lumen (20) extending between the distal end and the proximal end; and
a resilient push tab (46) coupled to the catheter adapter, wherein the resilient push tab comprises a first end (48) and a second end,
**CHARACTERIZED IN THAT**
the push tab further comprises an arced portion (52) between the first end and the second end, wherein the resilient push tab is configured to move from a raised position to a depressed position, wherein the arced portion is further from the catheter adapter when the resilient push tab is in the raised position than when the resilient push tab is in the depressed position, wherein in response to the resilient push tab being in the raised position, the first end and the second end are biased outwardly by the catheter adapter, wherein in response to the resilient push tab moving from the raised position to the depressed position, the first end and the second end are brought closer together.

10. The catheter assembly of claim 10, wherein the arced portion contacts the catheter adapter in response to the resilient push tab being moved from the raised position to the depressed position.

11. The catheter assembly of claim 10, wherein the catheter adapter comprises a side port (40) disposed between the distal end and the proximal end, wherein the resilient push tab is coupled to the catheter adapter proximal to the side port.

## Patentansprüche

1. Katheterbaugruppe (12), die aufweist:
einen Katheteradapter (14), der ein distales Ende (16), ein proximales Ende (18) und ein Lumen (20) aufweist, das sich zwischen dem distalen Ende und dem proximalen Ende erstreckt; und
eine Drücklasche (24), die aufweist:
ein erstes Ende (26);
ein zweites Ende (28) entgegengesetzt zu dem ersten Ende; und
eine Länge, die sich zwischen dem ersten Ende und dem zweiten Ende erstreckt;
**dadurch gekennzeichnet, dass**
die Drücklasche schwenkbar mit dem Katheteradapter gekoppelt ist, wobei die Drücklasche schwenkbar zwischen einer angehobenen Position und einer niedergedrückten Position bewegbar ist, wobei das erste Ende schwenkbar mit dem Katheteradapter gekoppelt ist, wobei das zweite Ende weiter von dem Katheteradapter entfernt ist, wenn die Drücklasche in der angehobenen Position ist, als wenn die Drücklasche in der niedergedrückten Position ist.

2. Katheterbaugruppe nach Anspruch 1, wobei das zweite Ende in einer ersten Position angeordnet ist, als Reaktion darauf, dass sich die Drücklasche in der angehobenen Position befindet, wobei das zweite Ende in einer zweiten Position angeordnet ist, als Reaktion darauf, dass sich die Drücklasche in der niedergedrückten Position befindet, wobei die zweite Position proximal zur ersten Position liegt.

3. Katheterbaugruppe nach Anspruch 1, wobei die Drücklasche zwei entgegengesetzte Vorsprünge (42a, b) aufweist, wobei der Katheteradapter zwei entgegengesetzte Vertiefungen (44a, b) aufweist, wobei die beiden entgegengesetzten Vorsprünge innerhalb der beiden entgegengesetzten Vertiefungen angeordnet sind, um die Drücklasche schwenkbar mit dem Katheteradapter zu koppeln.

4. Katheterbaugruppe nach Anspruch 1, wobei als Reaktion darauf, dass sich die Drücklasche in der angehobenen Position befindet, die Drücklasche im Allgemeinen senkrecht zu einer Längsachse (30) des Katheteradapters ist.

5. Katheterbaugruppe nach Anspruch 1, wobei als Reaktion darauf, dass sich die Drücklasche in der niedergedrückten Position befindet, die Drücklasche im Allgemeinen mit einer Längsachse (30) des Katheteradapters ausgerichtet ist.

6. Katheterbaugruppe nach Anspruch 1, wobei die Drücklasche eine Kante (32) aufweist, die so ausgebildet ist, dass sie den Katheteradapter als Reaktion darauf berührt, dass sich die Drücklasche schwenkbar von der niedergedrückten Position in die angehobene Position bewegt.

7. Katheterbaugruppe nach Anspruch 1, wobei eine proximale Oberfläche 34 der Drücklasche konkav ist.

8. Katheterbaugruppe nach Anspruch 1, wobei der Katheteradapter einen Seitenport (40) aufweist, der zwischen dem distalen Ende und dem proximalen Ende angeordnet ist, wobei die Drücklasche schwenkbar mit dem Katheteradapter proximal zum Seitenport gekoppelt ist.

9. Katheterbaugruppe (12), die aufweist:
einen Katheteradapter (14), der ein distales Ende (16), ein proximales Ende (18) und ein Lumen (20) aufweist, das sich zwischen dem distalen Ende und dem proximalen Ende erstreckt; und
eine elastische Drücklasche (46), die mit dem Katheteradapter gekoppelt ist, wobei die elastische Drücklasche ein erstes Ende (48) und ein zweites Ende aufweist,
**dadurch gekennzeichnet, dass**
die Drücklasche ferner einen gebogenen Abschnitt (52) zwischen dem ersten Ende und dem zweiten Ende aufweist, wobei die elastische Drücklasche so ausgebildet ist, dass sie sich von einer angehobenen Position in eine niedergedrückte Position bewegt, wobei der gebogene Abschnitt weiter von dem Katheteradapter entfernt ist, wenn sich die elastische Drücklasche in der angehobenen Position befindet, als wenn sich die elastische Drücklasche in der niedergedrückten Position befindet, wobei als Reaktion darauf, dass sich die elastische Drücklasche in der angehobenen Position befindet, das erste Ende und das zweite Ende durch den Katheteradapter nach außen vorgespannt sind, wobei als Reaktion darauf, dass sich die elastische Drücklasche von der angehobenen Position in die niedergedrückte Position bewegt, das erste Ende und das zweite Ende näher zusammengebracht werden.

10. Katheterbaugruppe nach Anspruch 10, wobei der gebogene Abschnitt den Katheteradapter als Reaktion darauf berührt, dass die elastische Drücklasche von der angehobenen Position in die niedergedrückte Position bewegt wird.

11. Katheterbaugruppe nach Anspruch 10, wobei der Katheteradapter einen Seitenport (40) aufweist, der zwischen dem distalen Ende und dem proximalen Ende angeordnet ist, wobei die elastische Drücklasche mit dem Katheteradapter proximal zum Seitenport gekoppelt ist.

## Revendications

1. Ensemble de cathéter (12), comprenant :
un adaptateur de cathéter (14), comprenant une extrémité distale (16), une extrémité proximale (18) et une lumière (20) s'étendant entre l'extrémité distale et l'extrémité proximale ; et
une languette de poussée (24) comprenant :
une première extrémité (26) ;
une seconde extrémité (28) opposée à la première extrémité ; et
une longueur s'étendant entre la première extrémité et la seconde extrémité ;
**CARACTÉRISÉ EN CE QUE**
la languette de poussée est couplée en pivotement à l'adaptateur de cathéter, dans lequel la languette de poussée est mobile en pivotement entre une position relevée et une position abaissée, dans lequel la première extrémité est couplée en pivotement à l'adaptateur de cathéter, dans lequel la seconde extrémité est plus éloignée de l'adaptateur de cathéter lorsque la languette de poussée est en position relevée que lorsque la languette de poussée est en position abaissée.

2. Ensemble de cathéter selon la revendication 1, dans lequel la seconde extrémité est disposée dans une première position en réponse au fait que la languette de poussée est en position relevée, dans lequel la seconde extrémité est disposée dans une seconde position en réponse au fait que la languette de poussée est en position abaissée, dans lequel la seconde position est proximale par rapport à la première position.

3. Ensemble de cathéter selon la revendication 1, dans lequel la languette de poussée comprend deux saillies opposées (42a, b), dans lequel l'adaptateur de cathéter comprend deux indentations opposées (44a, b), dans lequel les deux saillies opposées sont disposées à l'intérieur des deux indentations opposées pour coupler en pivotement la languette de poussée à l'adaptateur de cathéter.

4. Ensemble de cathéter selon la revendication 1, dans lequel, en réponse au fait que la languette de poussée est en position relevée, la languette de poussée est globalement perpendiculaire à un axe longitudinal (30) de l'adaptateur de cathéter.

5. Ensemble de cathéter selon la revendication 1, dans lequel, en réponse au fait que la languette de poussée est en position abaissée, la languette de poussée est globalement alignée avec un axe longitudinal (30) de l'adaptateur de cathéter.

6. Ensemble de cathéter selon la revendication 1, dans lequel la languette de poussée comprend un bord (32) configuré pour entrer en contact avec l'adaptateur de cathéter en réponse au déplacement pivotant de la languette de poussée de la position abaissée à la position relevée.

7. Ensemble de cathéter selon la revendication 1, dans lequel une surface proximale 34 de la languette de poussée est concave.

8. Ensemble de cathéter selon la revendication 1, dans lequel l'adaptateur de cathéter comprend un orifice latéral (40) disposé entre l'extrémité distale et l'extrémité proximale, dans lequel la languette de poussée est couplée en pivotement à l'adaptateur de cathéter à proximité de l'orifice latéral.

9. Ensemble de cathéter (12), comprenant :
un adaptateur de cathéter (14), comprenant une extrémité distale (16), une extrémité proximale (18) et une lumière (20) s'étendant entre l'extrémité distale et l'extrémité proximale ; et
une languette de poussée élastique (46) couplée à l'adaptateur de cathéter, dans lequel la languette de poussée élastique comprend une première extrémité (48) et une seconde extrémité,
**CARACTÉRISÉ EN CE QUE**
la languette de poussée comprend en outre une partie arquée (52) entre la première extrémité et la seconde extrémité, dans lequel la languette de poussée élastique est configurée pour se déplacer d'une position relevée à une position abaissée, dans lequel la partie arquée est plus éloignée de l'adaptateur de cathéter lorsque la languette de poussée élastique est en position relevée que lorsque la languette de poussée élastique est en position abaissée, dans lequel, en réponse au fait que la languette de poussée élastique est en position relevée, la première extrémité et la seconde extrémité sont sollicitées vers l'extérieur par l'adaptateur de cathéter, dans lequel, en réponse au déplacement de la languette de poussée élastique de la position relevée à la position abaissée, la première extrémité et la seconde extrémité sont rapprochées l'une de l'autre.

10. Ensemble de cathéter selon la revendication 10, dans lequel la partie arquée entre en contact avec l'adaptateur de cathéter en réponse au déplacement de la languette de poussée élastique de la position relevée à la position abaissée.

11. Ensemble de cathéter selon la revendication 10, dans lequel l'adaptateur de cathéter comprend un orifice latéral (40) disposé entre l'extrémité distale et l'extrémité proximale, dans lequel la languette de poussée élastique est couplée à l'adaptateur de cathéter à proximité de l'orifice latéral.
